# FASCICULE DE BREVET EUROPEEN

(11) **EP 0 602 488 B1**
(45) Date de publication et mention de la délivrance du brevet: **31.03.1999**
(21) Numéro de dépôt: 93119597.8
(22) Date de dépôt: 06.12.1993
(51) Int. Cl.: C07F 15/00, C07F 15/02, C12Q 1/26, C12Q 1/54, C12Q 1/00, G01N 27/30, C07D 213/06, C07D 213/89, C07D 213/38

(54) **Complexes d'un métal de transition à ligands 2,2-bipyridine substitués par au moins un radical ammonium alkyle, leur procédé de fabrication et leur application comme médiateur redox**
Komplexe eines Übergangsmetalls mit 2,2-Bipyridin-Liganden, die mit mindestens einer Ammoniumalkylgruppe substituiert sind, Verfahren zu ihrer Herstellung und ihre Anwendung als Redoxvermittler
Complexes of a transition metal with 2,2-bipyridine ligands substituted by at least one ammonium alkyl radical, process for their fabrication and their use as redox mediator

(30) Priorité: 15.12.1992 FR 9215214
(43) Date de publication de la demande: 22.06.1994
(73) Titulaire: ASULAB S.A., CH-2501 Bienne (CH)
(72) Inventeur: Fraser, David, CH-1260 Nyon (CH); Zakeeruddin, Shaik Mohammed, CH-1020 Renens (CH); Graetzel, Michael, CH-1025 St-Sulpice (CH)
(74) Mandataire: Caron, Gérard

(56) Documents cités:
- WO-A-87/06706
- WO-A-92/12254
- WO-A-92/14741
- WO-A-92/14836
- CHEMICAL ABSTRACTS, vol. 111, no. 14, 2 Octobre 1989, Columbus, Ohio, US; abstract no. 125629d, V.E. MAIER ET AL. & IZV. AKAD. NAUK SSSR, SER. KHIM., vol.3, 1989 pages 700 - 703
- CHEMICAL ABSTRACTS, vol. 113, no. 12, 17 Septembre 1990, Columbus, Ohio, US; abstract no. 98457d, A.J. DOWNARD ET AL. & J. PHYS. CHEM., vol.94, no.17, 1990 pages 6754 - 6764
- CHEMICAL ABSTRACTS, vol. 109, no. 12, 19 Septembre 1988, Columbus, Ohio, US; abstract no. 100637a, F. DAIRE ET AL. & ELECTROCHIM. ACTA, vol.33, no.4, 1988 pages 567 - 571
- CHEMICAL ABSTRACTS, vol. 116, no. 20, 18 Mai 1992, Columbus, Ohio, US; abstract no. 203134h, C.P. HORWITZ ET AL. & INORG. CHEM., vol.31, no.9, 1992 pages 1607 - 1613
- CHEMICAL ABSTRACTS, vol. 116, no. 18, 4 Mai 1992, Columbus, Ohio, US; abstract no. 186612t, M.A. HAYES ET AL. & J. CHEM. SOC., DALTON TRANS., vol.4, 1992 pages 703 - 708
- CHEMICAL ABSTRACTS, vol. 104, no. 4, 27 Janvier 1986, Columbus, Ohio, US; abstract no. 26689x, G. JONES II ET AL. & J. ORG. CHEM., vol.50, no.26, 1985 pages 5776 - 5782
- CHEMICAL ABSTRACTS, vol. 101, no. 18, 29 Octobre 1984, Columbus, Ohio, US; abstract no. 159978a, S. SAHAMI ET AL. & REPORT 1984 - Dep. Chem. State Univ. New York, Buffalo, NY Order No. AD-A139 170 & GOV. REP. ANNOUNCE. INDEX., vol.84, no.13, 1984 page 74 U.S.
- CHEMICAL ABSTRACTS, vol. 109, no. 26, 26 Décembre 1988, Columbus, Ohio, US; abstract no. 239126b, E. GARCIA ET AL. & INORG. CHEM., vol.27, no.24, 1988 pages 4377 - 4382
- CHEMICAL ABSTRACTS, vol. 105, no. 16, 20 Octobre 1986, Columbus, Ohio, US; abstract no. 141913y, F. DAIRE ET AL. & J. ELECTROANAL. CHEM. INTERFACIAL ELECTROCHEM., vol.205, no.1-2, 1986 pages 309 - 318
- DATABASE WPI Week 8930, Derwent Publications Ltd., London, GB; AN 89-217094 & JP-A-1 155 244 (RIKAGAKU KENKYUSHO - TERUMO CORP.)

## Description

La présente invention concerne une nouvelle famille de complexes d'un métal de transition du groupe (VIII) tel que le fer (II), le ruthénium (II), ou l'osmimium (II), comportant trois ligands bidentés 2,2'-bipyridine, dont un ligand au moins est substitué par au moins un groupe quaternaire ammonium alkyle, ainsi que leurs sels.

L'invention concerne aussi un procédé de synthèse de ces nouveaux complexes.

A titre d'exemple d'application, l'invention concerne également l'utilisation de ces composés en tant que médiateurs dans les réactions redox. Ces complexes se sont plus particulièrement révélés intéressants dans les mesures de concentration d'un composant en solution, et notamment du glucose dans un liquide biologique ou physiologique, en agissant comme médiateurs pour le transfert d'électrons entre une enzyme spécifique dudit composant, tel que la glucose oxydase (GOD) dans le cas du glucose, et une électrode de mesure dans un capteur ampérométrique.

D'après R. SZENTRIMAY and al. (Electrochemical studies of biological systems - ch. 9 page 143-169 - Am. Chem. Soc. Washington D.C. 1977), qui a établi la liste des critères vers lesquels doit tendre un médiateur idéal, on sait qu'un médiateur doit notamment posséder un potentiel normal d'oxydo réduction Eₒ bien déterminé dans les conditions expérimentales, et une vitesse de transfert des électrons k_{med} assez rapide. Dans le cas du dosage du glucose, on souhaite en particulier avoir un médiateur ayant une constante k_{med} supérieure à 1 x 10⁶, M⁻¹ s⁻¹ et un potentiel normal d'oxydo réduction Eₒ le plus bas possible, de préférence compris entre -400 mV et +400 mV pour réduire ou supprimer les risques d'interférence avec d'autres composés présents dans la solution à doser.

De nombreux composés ont déjà été proposés comme médiateurs dans les réactions redox tel que le ferrocene et ses dérivés [Cass A-E-G et coll. Anal.Chem. 56, 667-671 (1984)]. On a également proposé comme indicateur d'oxydoréduction des composés de type viologène, tel que [Ru(bpy)₂L]Cl₄ dans lequel L est une 5-méthyl -5'[2 ou 5 (4-méthyl-4,4' bipyridinium) éthyl ou pentyl]-2,2'-bipyridine. (Chemical Abstracts, vol. 111, 1989, 125629d). Plus récemment la demande internationale WO 92/14741 au nom de la demanderesse divulgue une famille de complexes mono, bis ou tris (2,2'-bipyridine substituée) d'un métal choisi parmi le fer, le ruthénium, l'osmium ou du vanadium; dans cette famille le choix particulier d'un métal de transition et de substituants donneurs d'électrons sur les ligands a permis d'influencer favorablement la stabilité du médiateur, la constante k_{med} de vitesse de médiation et le potentiel normal Eₒ d'oxydoréduction.

La nouvelle famille de médiateurs selon l'invention permet d'avoir une constante de vitesse de médiation k_{med} encore plus favorable, tout en conservant un potentiel normal d'oxydo réduction Eₒ compris dans les limites souhaitées pour un médiateur idéal. Dans le cas où les ligands sont des ligands bidentés 2,2'-bipyridine les composés préférés selon l'invention répondent à la formule générale (I) suivante : 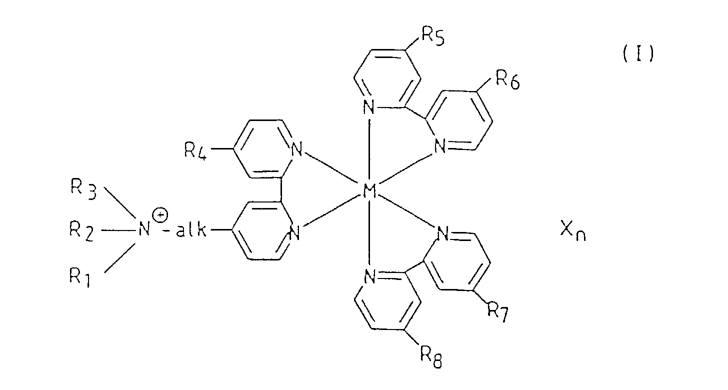 dans laquelle R₁, R₂ et R_{3,} qui peuvent être identiques ou différents, représentent séparément un radical alkyle linéaire ou ramifié de 1 à 5 atomes de carbone, ou représentent ensemble avec l'atome d'azote auquel ils sont liés un radical hétérocyclique ayant 5 à 7 atomes de carbone; "alk" représente un radical alkylène linéaire ou ramifié ayant 1 à 5 atomes de carbone; R₄ à R_{8,} qui peuvent être identiques ou différents, représentent l'hydrogène, un groupe hydroxy, alkoxy, aryloxy, amino-primaire secondaire ou tertiaire, ou le groupe -alk-N⁺(R₁R₂R₃) dans lequel "alk", R₁, R₂ et R₃ ont les significations précédentes; M représente un métal de transition tel que le fer, le ruthénium, ou l'osmium; X représente un anion tel que Cl⁻, PF₆⁻, Br⁻, BF₄⁻ et n un nombre entier correspondant au nombre total de charges positives des ligands et du métal de transition.

A titre de composés chimiques nouveaux, utiles notamment par la synthèse des composés de formule I, l'invention concerne aussi les bipyridines substituées par au moins un radical ammonium alkyle de formule générale (II) dans laquelle R₁, R₂, R₃, R₄ et "alk" 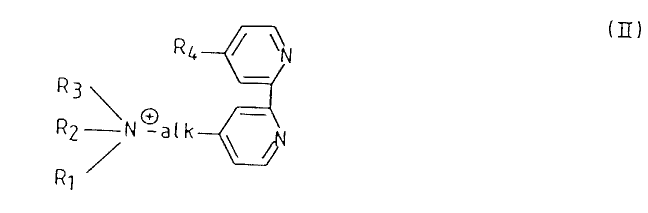 ont les mêmes significations que celles indiquées pour la formule générale I.

Selon une réalisation préférée, l'invention concerne des composés dans lesquels le radical alkylène "alk" représente le radical méthylène -CH₂-.

Selon une autre réalisation préférée, l'invention concerne les composés dans lesquels R₄ représente N(R₁R₂R₃).

Selon une autre réalisation préférée l'invention concerne des composés dans lesquels le radical "alk" et R₄ ont les significations précédentes et R₁, R₂ et R₃ représentent le radical éthyle. Les composés de la présente invention possèdent alors au moins un ligand 4,4'-bis (triethylammonium-méthyl)-2,2' bipyridine et répondent à la formule générale III 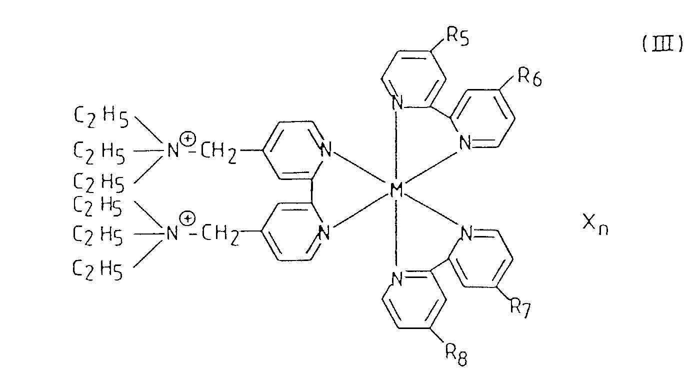 dans laquelle R₅, R₆, R₇, R₈, X et n ont les mêmes significations que celles indiquées pour la formule générale I.

Les complexes préférés de la présente invention répondant à la formule générale III sont :
- le complexe bis[4,4'-bisamino-2,2'bipyridine]mono[4,4'-bis(triéthylammoniumméthyl)-2,2' bipyridine] d'Osmium, désigné par la suite Os(DA-bpy)₂(TEAM-bpy), et ses sels.
- le complexe bis[4,4'-bisamino-2,2'-bipyridine] mono[4,4'-bis(triethylammoniummethyl)-2,2'bipyridine] de ruthénium, désigné par la suite Ru(DA-bpy)₂(TEAM-bpy) et ses sels.
- le complexe bis[4,4'-bis(diméthylamino)-2.2'bipyridine] mono[4,4'-bis(triéthylaminométhyl)-2,2'-bipyridine] de ruthénium, désigné par la suite Ru(DMA-bpy)₂(TEAM-bpy) et ses sels.
- le complexe bis [4,4'-bis(triéthylammoniumméthyl)2,2'bipyridine]mono(4,4'-bisamino)2,2'-bipyridine d'Osmium, désigné par la suite Os(TEAM-bpy)₂(DA-bpy) et ses sels.
- le complexe tris[4,4'-bis(triéthylammoniumméthyl)-2,2'-bipyridine] d'Osmium, désigné par la suite Os(TEAM-bpy)_{3,} et ses sels.
- le complexe tris[4,4'-bis(triéthylammoniumméthyl)-2,2'-bipyridine] de fer, désigné par la suite Fe(TEAM-bpy)₃, et ses sels.

Selon une autre réalisation préférée, l'invention concerne des composés dans lesquels le radical "alk" et R₄ ont les significations précédentes et R₁, R₂ et R₃ forment ensemble avec l'atome d'azote auquel ils sont liés le radical N-pyridyl. Les composés de la présente invention possèdent alors au moins un ligand 4,4'-bis(N-pyridiniumméthyl)-2,2'bipyridine et répondent à la formule générale IV 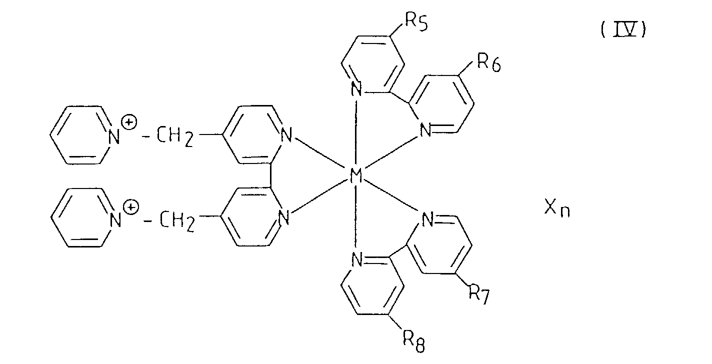 dans laquelle R₅, R₆, R₇, R₈, X et n ont les mêmes significations que celles indiquées pour la formule I.

Les complexes préférés de la présente invention répondant à la formule générale IV sont :
- le complexe bis[4,4'-bisamino-2,2'-bipyridine]-mono[4,4'-bis(N-pyridiniumméthyl)-2,2' bipyridine] d'Osmium, désigné par la suite Os(DA-bpy)₂(NPM-bpy) et ses sels.
- le complexe bis[4,4'-bisamino-2,2'-bipyridine]-mono[4,4'-bis(N-pyridiniumméthyl)2,2'-bipyridine] de ruthénium, désigné par la suite Ru(DA-bpy)₂(NPM-bpy) et ses sels.
- le complexe bis[4,4'-bis(N-pyridiniumméthyl)-2,2'bipyridine mono(4,4'-bisamino-2,2'-bipyridine) d'Osmium, désigné par la suite Os(NPM-bpy)₂(DA-bpy) et ses sels.
- le complexe tris[4,4'-bis(N-pyridiniumméthyl)-2,2'-bipyridine] d'Osmium, désigné par la suite Os(NPM-bpy)₃ et ses sels.
- le complexe tris[4,4'-bis(N-pyridiniuminéthyl)-2,2'-bipyridine] de fer, désigné par la suite Fe(TEAM-bpy)₃ et ses sels.

Selon une autre réalisation préférée, l'invention concerne des composés de formule (I), (III) ou (IV) dans lesquels le métal M de transition est l'Osmium.

Les composés de formules générales I, II, III ou IV correspondent aux composés préférés de l'invention, dans lesquels les ligands bipyridine sont substitués en position 4,4', mais il est bien évident que les composés substitués en d'autres positions font également partie de l'invention.

Lorsque les composés de la présente invention sont utilisés en tant que médiateurs redox on choisira de préférence un sel soluble tel que le Chlorure ou l'hexafluorophosphate.

Le procédé général pour obtenir les composés de l'invention correspondant aux formules générales I, III ou IV consiste, dans une première étape à préparer un composé de formule II ayant un ligand substitué par un radical ammonium quaternaire en faisant réagir une 4,4'-bis-bromoalkyl-2,2'-bipyridine sur une amine tertiaire en excès de formule N(R₁R₂R₃), dans laquelle R₁, R₂ et R₃ ont les significations données pour la formule (I), en présence d'un solvant organique approprié en chauffant à reflux de solvant sous atmosphère d'azote pendant environ 3 heures et à isoler le composé de formule II obtenu par les moyens habituels; puis dans une deuxième étape à faire réagir le composé de formule (II) avec une quantité sensiblement stoechiométrique d'un sel soluble du complexe formé par le métal M et deux ligands bipyridine respectivement substitués par R_{5,} R₆, R₇ et R₈, ledit sel ayant été préalablement préparé selon des méthodes connues.

Dans le cas où les substituants R₄ à R₆ représentent -N(R₁R₂R₃), on prépare d'abord le complexe Bis d'un composé de formule (II) avec un métal approprié M, puis on fait réagir le composé obtenu avec une bipyridine convenablement substituée par R₇ et R₈ conformément à la deuxième étape du procédé général.

Dans le cas où les substituants R₄ à R₈ représentent -N(R₁R₂R₃) le procédé consiste à chauffer à reflux de solvant une solution contenant dans des proportions sensiblement stoechiométriques le composé de formule (II) préparé dans la première étape et un sel soluble du métal M.

Pour tous les composés de la présente invention, la présence d'au moins une charge positive permanente sur au moins un ligand bipyridine permet d'accroître la charge globale positive totale des complexes jusqu'à des valeurs qu'il n'est pas encore possible d'atteindre avec les médiateurs antérieurement connus.

L'invention sera mieux comprise en référence aux exemples donnés ci-après pour illustrer la préparation et les propriétés électrochimiques d'un nombre représentatif de composés de l'invention en vue d'une application comme médiateurs dans les réactions redox permettant le titrage d'un composé en solution en présence d'une enzyme oxydoreductase appropriée.

### Exemple 1 - Préparation du complexe [Os(DA-bpy)₂(TEAM-bpy)](PF₆)₄

Dans une première étape on a préparé le bromure de 4,4'-bis(triéthylammoniumméthyl)-2,2'-bipyridine (désigné par la suite TEAM-bpy). Dans un réacteur on a dissous 4g(11,7 mmol) de 4,4'-bromométhylbipyridine) dans 16 ml de chloroforme. A cette solution on a ajouté 6 ml de triéthylamine (59,4 mmol en excès) et on a chauffé le mélange à reflux pendant au moins 3 heures à environ 45 - 50 °C sous atmosphère d'azote à pression atmosphérique. On a isolé le produit par filtration, lavé au chloroforme et séché sous vide poussé. Le produit obtenu a pour formule brute C₂₄H₄₀N₄Br₂(3,03H₂0) et pour analyse élémentaire :

| | C% | H% | N% | H₂0 |
|---|---|---|---|---|
| calculé | 48,1 | 7,75 | 9,35 | 9,11 |
| trouvé | 46,51 | 7,49 | 9,32 | 9,12 |

L'analyse RMN dans une solution de D₂0 correspond au spectre suivant :
1,5(t); 3,4(q); 4,65 (s); 7,8(dd); 8,3(d); 8,9(d)

Dans une deuxième étape on a dissous 0,053 g (0,097 m mol) de TEAM-bpy dans 0,5 ml d'eau, puis on a ajouté 10 ml d'éthyléneglycol et 0,05 g (0,075 mmol) de complexe bis(4,4'-bisamino-2,2'bipyridine)Cl₂ d'osmium (désigné par la suite 0s(DA-bpy)₂Cl₂ et chauffé le mélange à reflux pendant au moins 4 heures à environ 140-150 °C sous atmosphère d'azote à pression atmosphérique jusqu'à apparition d'une couleur brune. Après refroidissement à température ambiante, on a versé le milieu réactionnel dans une ampoule à décanter et précipité le produit de l'éthylène glycol en ajoutant 20 ml de diéthyléther et 5 ml d'acétone, en éliminant la phase éther et en répétant l'opération jusqu'à précipitation visible du composé du titre. On a isolé le complexe par filtration et on l'a dissous dans 10 ml d'eau; par addition d'une solution aqueuse d'hexafluorophosphate de potassium on a précipité le complexe sous forme de son sel hexafluorophosphate; on l'a isolé par filtration, lavé à l'eau, puis au diéthyléther et séché sous vide poussé.

### Exemple 2 - Préparation du complexe [Ru(DA-bpy)₂(NPM-bpy)](PF₆)₄

Dans une première étape on a préparé le composé 4,4'-bis(N-pyridiniumméthyl)-2,2'-bipyridine (désigné par la suite NPM-bpy). Dans un réacteur on a dissous 4 g (11,7 mmol) de 4,4'-bromoéthylbipyridine dans 15 ml de chloroforme. A cette solution on a ajouté 6 ml (75 mmol en excès) de pyridine et on a chauffé le mélange à reflux pendant au moins 3 heures à environ 45 - 50 °C sous atmosphère d'azote à la pression atmosphérique. On a isolé le produit par filtration, lavé au chloroforme et séché sous vide poussé. L'analyse RMN du composé obtenu dans une solution de D₂0 correspond au spectre suivant :
6,1(s); 7,55(dd); 8,15 (d); 8,25(d); 8,65(t); 8,75(8D); 9,05(dd)

Dans une deuxième étape on a dissous 0,028 g (0,050 m mol) de NPM-bpy dans 5 ml d'eau, puis on a ajouté 10 ml de diméthylformamide et 0,025 g (0,043 mmol) de complexe bis(4,4'-bisamino-2,2'bipyridine)Cl₂ de ruthénium (désigné par la suite Ru(DA-bpy)₂Cl₂) et chauffé le mélange à reflux pendant au moins 4 heures à environ 140 - 150 °C sous atmosphère d'azote, à pression atmosphérique. Après avoir laissé refroidir à température ambiante, on a filtré la solution et concentré le solvant au quart du volume d'origine. On a ensuite fait précipiter le complexe par addition de diéthyléther, isolé le complexe par filtration et dissous dans 10 ml d'eau; par addition d'une solution aqueuse d'hexafluorophosphate de potassium on a précipité le complexe sous forme de son sel hexafluorophosphate; on l'a isolé par filtration, lavé au diéthyléther et séché sous vide poussé.

### Exemple 3 - Préparation du complexe [Os(TEAM-bpy)₂(DA-bpy)](PF₆)₆

Dans un réacteur on a dissous dans 1 ml l'eau 0,221 g(0,406 mmol) de TEAM-bpy, préparé comme indiqué dans la première étape de l'exemple 1, puis on a ajouté 10 ml d'éthyleneglycol et ensuite 0,10 g (0,203 mmol) de sel K₂OsCl₆ et chauffé le mélange à reflux pendant au moins 1 heure et demi sous atmosphère d'azote, à pression atmosphérique à environ 140 - 150° C. Puis, après avoir précipité, isolé et remis le complexe en solution on a ajouté 0,042 g (0,225 mmol) de 4,4'-bisamino-2,2'bipyridine et chauffé le milieu réactionnel à reflux à environ 140 - 150° C pendant au moins 1 heure et demie sous atmosphère d'azote à la pression atmosphérique jusqu'à apparition d'une couleur brune. Le complexe obtenu a ensuite été précipité, isolé, lavé et séché comme indiqué dans l'exemple 1.

### Exemple 4 - Préparation du complexe [Os(NPM-bpy)₂(DA-bpy)](PF₆)₆

Le composé du titre a été obtenu en suivant le procédé décrit dans l'exemple 1, mais en portant de NPM-bpy, tel que préparé dans la première étape de l'exemple 2.

### Exemple 5 - Préparation du complexe [Os(TEAM-bpy)₃](PF₆)₈

Dans un réacteur on a dissous 0,183 g (0,337 m mol) de TEAM-bpy, préparé comme indiqué dans la première étape de l'exemple 1, dans 1 ml d'eau, puis on a ajouté 10 ml d'éthylèneglycol et ensuite 0,05 g (0,101 m mol) de K₂OsCl₆ et chauffé le mélange à reflux à environ 140 - 150 °C pendant au moins 5 heures, sous atmosphère d'azote et à pression atmosphérique, jusqu'à apparition d'une couleur brune. Après refroidissement on a précipité le produit du titre de l'éthylèneglycol par la méthode décrite dans la deuxième étape de l'exemple 1. Après isolation du complexe sous forme d'hexafluorophate on obtient un composé ayant pour formule brute :
C₇₂ H₁₂₀ N₁₂ Os P₆ F₄₈
et pour analyse élémentaire

| | C % | H % | N % |
|---|---|---|---|
| calculé | 34,54 | 4,83 | 6,71 |
| trouvé | 38,44 | 4,16 | 6,48 |

### Exemple 6 - Préparation des complexes [Ru (DMA-boy)₂(TEAM-bpy)](PF₆)₄ et [Ru (DA-bpy)₂(TEAM-bpy)](PF₆)₄

En suivant le procédé décrit dans l'exemple 2, mais en partant de TEAM-bpy et de Ru(4,4'-diméthylamino-2,2'-bipyridine)Cl₂ on a obtenu le complexe [Ru(DMA-bpy)₂(TEAM-bpy)](PF₆)₄; de même en partant de TEAM-bpy et de Ru(DA-bpy)₂Cl₂, on a obtenu le complexe [Ru(DA-bpy)₂(TEAM-bpy)](PF₆)₄.

### Exemple 7 - Préparation du complexe [Os(DA-bpy)₂(NPM-bpy)](PF₆)₄

Le composé du titre a été obtenu en suivant le procédé décrit dans l'exemple 1, mais en partant de NPM-bpy, tel que préparé dans la première étape de l'exemple 2, et de Os(DA-bpy)₂Cl₂.

### Exemple 8 - Préparation de [Os(NPM-bpy)₃](PF₆)₈ et [Fe(TEAM-bpy)₃](PF₆)₈

En suivant le procédé décrit dans l'exemple 5 mais en partant de NPM-bpy on a obtenu le complexe [Os(NPM-bpy)₃](PF₆)₈; de même en partant de TEAM-bpy en présence de FeCl₂,4H₂0 on a obtenu [Fe(TEAM-bpy)₃](PF₆)₈ et [Fe(NPM-bpy)₃] (PF₆)₈.

Les complexes des exemples 1 à 8 ont en outre été caractérisés par leur spectre d'absorption en lumière UV et visible qui présente la bande spécifique des liaisons métal-ligand aux environs de 500 nm. Le tableau ci-après donne pour chacun des composés la longueur d'onde du maximum et la valeur du coefficient d'extinction.

**Tableau I**

| Complexe | λmax (nm) | ε (M⁻¹cm⁻¹) |
|---|---|---|
| [Os(DA-bpy)₂(TEAM-bpy)](PF₆)₄ | 556 | 8743 |
| [Os(DA-bpy)₂(NPM-bpy)](PF₆)₄ | 536 | 10818 |
| [Os(TEAM-bpy)₂(DA-bpy)](PF₆)₆ | 512 | 9428 |
| [Os(NPM-bpy)₂(DA-bpy)](PF₆)₆ | 516 | 11054 |
| [Os(TEAM-bpy)₃](PF₆)₈ | 492 | 7364 |
| [Os(NPM-bpy)₃](PF₆)₈ | 494 | 10449 |
| [Ru(DA-bpy)₂(TEAM-bpy)](PF₆)₄ | 526 | 10559 |
| [Ru(DA-bpy)₂(NPM-bpy)](PF₆)₄ | 530 | 8593 |
| [Ru(DMA-bpy)₂(TEAM-bpy)](PF₆)₄ | 506 | 9296 |
| [Fe(TEAM-bpy)₃](PF₆)₈ | 534 | 17375 |
| [Fe(NPM-bpy)₃](PF₆)₈ | 533 | 9845 |

L'activité potentiodynamique des composés de l'invention, et leur aptitude à agir comme médiateur rédox ont été évaluées par des méthodes usuelles de voltamétrie cyclique permettant de déterminer le potentiel normal d'oxydoréduction Eₒ la constante k_{med} et le comportement électrochimique du complexe.

Dans une première méthode on a dissous le complexe (5.10⁻⁴ M) dans un solvant organique (acétonitrile contenant LiCl0₄ 0,2M), ou dans un tampon phosphate PBS (NaCl 50 mM, NaH₂PO₄ 5 mM ajusté à pH 7,4), puis on a effectué un balayage à vitesse constante (25 mV.s-1) en utilisant une électrode en carbone vitreux comme électrode de travail et une électrode au calomel (SCE) comme électrode de référence, en maintenant la cellule de mesure sous courant d'azote.

Dans une deuxième méthode, plus particulièrement destinée à mettre en évidence les propriétés de médiateur des complexes pour le dosage de glucose en présence d'une enzyme oxydase GOD, on a utilisé le tampon phosphate PBS en remplaçant l'électrode de travail en carbone vitreux par une électrode en graphite spectroscopique sur laquelle on a adsorbé le complexe à étudier.

On a effectué les mêmes mesures en utilisant comme composé de référence le complexe bis(4,4'-bisamino-2,2'-bipyridine)mono(4,4'diméthyl-2,2'-bipyridine) d'osmium ou de ruthénium (désigné par la suite Os(DA-bpy)₂(dm-bpy) et Ru( DA-bpy)₂(dm-bpy); ces complexes ont en effet des structures proches de celles des complexes de l'invention mais ne comportent aucun substituant ammonium quaternaire.

Ces mesures ont permis de déterminer le potentiel normal d'oxydo-reduction Eₒ, dans différentes conditions, et la constante de vitesse de médiation k_{med}.

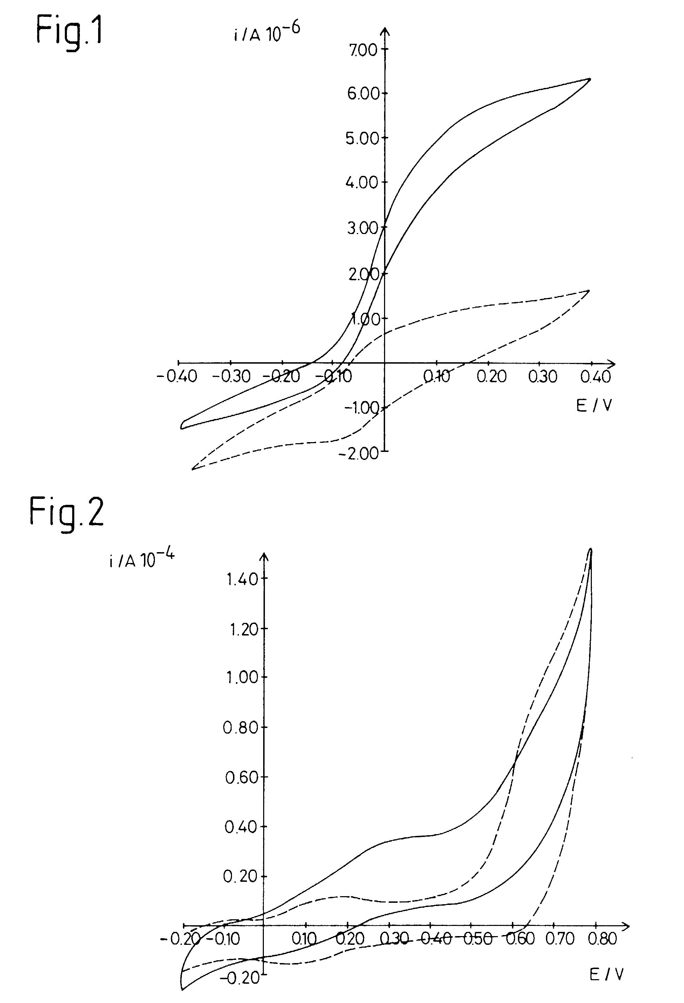

Ces mesures sont rapportées ci-après de façon plus détaillée en référence au complexe [Os(DA-bpy)₂(TEAM-bpy](PF₆)₄ pris à titre d'exemple. Dans un solvant organique on a obtenu pour valeur du potentiel d'oxydoréduction E°_{CH3CN} = 240 mV. Dans un tampon phosphate PBS on a obtenu un potentiel d'oxydo-réduction de E°_{PBS} = -50 mV et un voltamogramme correspondant à la figure 1. Les mesures cinétiques effectuées correspondent à une constante k_{med} = 1,5 · 10⁷ M⁻¹ s⁻¹ (selon le protocole R.S Nicholson and I. Shain, Anal.Chem., 76 (1964) 706).

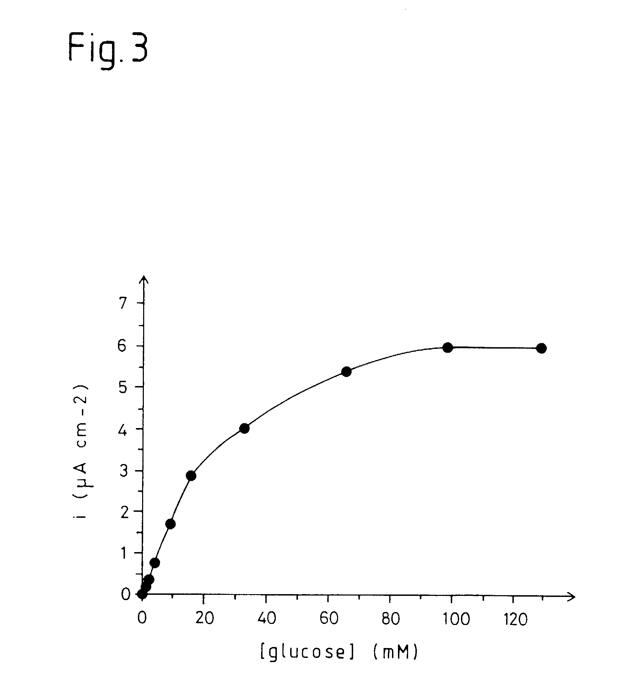

En effectuant les mêmes mesures dans un tampon phosphate PBS mais après adsorption sur une électrode de graphite spectroscopique on obtient le voltamogramme de la figure 2 dans laquelle la courbe en trait pointillé correspond aux mesures effectuées en présence de glucose (100 mM) et la courbe en trait plein en absence de glucose. Après adsorption du médiateur son potentiel redox devient positif, comme cela est souvent observé et correspond à la valeur E°_{ads} = 180 mV. En dernier lieu la figure 3 représente à titre illustratif la courbe obtenue lorsqu'on applique un potentiel constant de 200 mV et qu'on fait varier la concentration de glucose. Cette courbe monte qu'on obtient une excellente linéarité jusqu'à presque 20 mM.

Les mesures effectuées avec d'autres composés de l'invention sont rapportées dans le tableau II suivant, comparativement aux deux composés de référence précités

**Tableau II**

| Complexe | E°CH_{₃CN} (mV) | E°_{PBS} (mV) | E°_{ads} (mV) | k_{med} (M⁻¹s⁻¹) |
|---|---|---|---|---|
| [Os(DA-bpy)₂(TEAM-bpy)] (PF₆)₄ | 240 | -50 | 180 | 1,5.10⁷ |
| [Os(DA-bpy)₂(NPM-bpy)](PF₆)₄ | 170 | -50 | 55 | 1,4.10⁷ |
| [Os(TEAM-bpy)₂(DA-bpy)](PF₆)₆ | 400 | 270 | 430 | 4,0.10⁵ |
| [Os(NPM-bpy)₂(DA-bpy)](PF₆)₆ | 350 | 300 | 650 | 1,0.10⁶ |
| [Os(TEAM-bpy)₃] (PF₆)₈ | 790 | 610 | 655 | 1,0.10⁵ |
| [Os(NPM-bpy)₃](PF₆)₈ | 790 | 630 | n.d. | n.d. |
| [Ru(DA-bpy)₂(TEAM-bpy)](PF₆)₄ | 680 | 550 | 555 | 3,0.10⁶ |
| [Ru(DA-bpy)₂(NPM-bpy)](PF₆)₄ | 670 | 530 | 530 | 3,7.10⁶ |
| [Ru(DMA-bpy)₂(TEAM-bpy)](PF₆)₄ | 510 | 410 | 460 | 1,3.10⁶ |
| [Fe(TEAM-bpy)₃](PF₆)₈ | 1170 | 1180 | n.d. | n.d. |
| [Fe(NPM-bpy)₃] (PF₆)₈ | 1075 | 975 | n.d. | n.d. |
| [Os(DA-bpy)₂(dm-bpy)]Cl₂ | 120 | -60 | 65 | 6,7.10⁶ |
| [Ru(DA-bpy)₂(dm-bpy)]Cl₂ | 600 | 410 | 450 | 1,0.10⁶ |

Ces résultats montrent que les composés de l'invention possédant un ligand substituée par un ammonium quaternaire, c'est-à-dire porteur d'une charge positive globale de +5 ont une vitesse de médiation très supérieure à celle des homologues les plus proches non substitués, porteur d'une charge globale de +3. Par exemple le complexe Os(DA-bpy)₂(TEAM-bpy) possède une constante k_{med}=1,5.10⁷M⁻¹s⁻¹ , environ 2 fois plus élevée que celle de Os(DA-bpy)₂(dm-bpy) et pour Ru(DA-bpy)₂(dm-bpy) il existe encore un facteur 3 entre les deux constantes k_{med} de vitesse de médiation. Pour tous ces composés le potentiel d'oxydoréduction est assez bas. Pour les complexes Os(DA-bpy)₂(TEAM-bpy) et Os(DA-bpy)₂(NPM-bpy) on observe aussi que les constantes k_{med} et Eₒ ont pratiquement la même valeur : la nature du substituant ammonium quaternaire (TEAM ou NPM) n'influence pas notablement les propriétés du complexe. Les complexes ayant une charge globale positive maximum, - par exemple +9 pour Os(TEAM-bpy)₃ -, ont des valeurs de constantes un peu moins favorables pour l'application comme médiateur pour le dosage du glucose en présence de glucose oxydase; ces complexes peuvent toutefois présenter un intérêt comme médiateurs pour le dosage d'un composant en solution autre que le glucose en présence d'une autre enzyme spécifique ayant des caractéristiques différentes de celles de la glucose oxydose GOD.

Cet ensemble de propriétés permet donc d'utiliser les complexes selon l'invention en tant que médiateurs redox pour le dosage d'un composant en solution en présence d'une enzyme spécifique dudit composant. Ils se sont révélés particulièrement intéressants pour le dosage du glucose en présence d'enzyme glucose oxydase (GOD).

## Revendications

1. Complexe d'un métal de transition ayant trois ligands bidentés dont au moins un est substitué en position 4,4' par au moins un groupe quaternaire ammonium alkyle caractérisé en ce qu'il correspond à la formule générale I 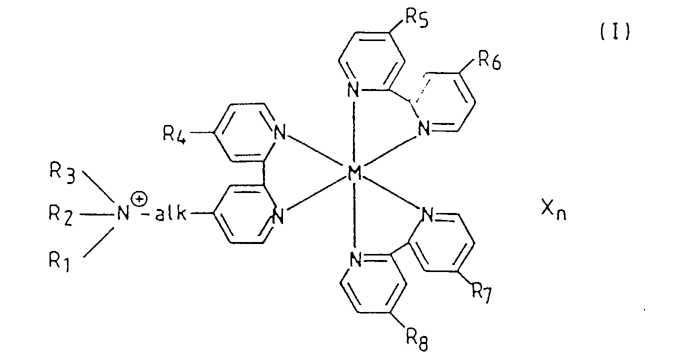 dans laquelle R₁, R₂ et R₃ qui peuvent être identiques ou différents, représentent séparément un radical alkyle linéaire ou ramifié de 1 à 5 atomes de carbone, ou représentent ensemble avec l'atome d'azote auquel ils sont liés un radical hétérocyclique ayant 5 à 7 atomes de carbone; "alk" représente un radical alkylène linéaire ou ramifié ayant 1 à 5 atomes de carbone; R₄ à R₈, qui peuvent être identiques ou différents, représentent l'hydrogène, un groupe hydroxy, alkoxy, aryloxy, amino-primaire secondaire ou tertiaire, ou le groupe -alk-N± (R₁R₂R₃) dans lequel "alk", R₁, R₂ et R₃ ont les significations précédentes; M représente un métal de transition tel que le fer, le ruthénium, ou l'osmium; X représente un anion tel que Cl⁻, PF₆⁻, Br⁻, BF₄⁻ et n un nombre entier correspondant au nombre de charges positives des ligands et du métal de transition.

2. Complexe selon la revendication 2, caractérisé en ce que le radical alkylène "alk" représente le radical méthylène - CH₂-.

3. Complexe selon les revendication 1 et 2 caractérisé en ce que R4 représente le groupe -N⁺(R₁R₂R₃).

4. Complexe selon les revendications 1 à 3, caractérisé en ce que -N⁺(R₁R₂R₃) représente le groupe triéthylammonium, et correspond à la formule générale III 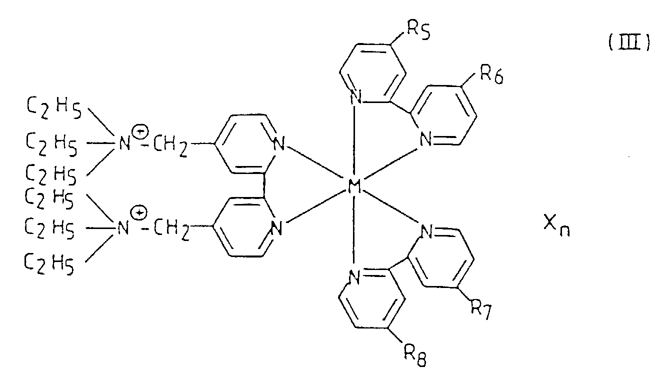 dans laquelle R₅, R₆, R₇, R₈ X et n ont les mêmes significations que celles indiquées pour la formule générale I.

5. Complexe selon la revendication 4, caractérisé en ce qu'il correspond à l'un des composés de formule
- bis[4,4'-bisamino-2,2'bipyridine]mono [4,4'-bis(triéthylammoniumméthyl)-2,2' bipyridine] d'Osmium, désigné par la suite Os(DA-bpy)₂(TEAM-bpy), et ses sels.
- bis[4,4'-bisamino-2,2'-bipyridine] mono [4,4'-bis (triethylammoniummethyl)-2,2'bipyridine] de ruthénium, désigné par la suite Ru(DA-bpy)₂(TEAM-bpy) et ses sels.
- bis[4,4'-bis(diméthylamino)-2,2'bipyridine] mono[4,4'-bis(triéthylaminométhyl)-2,2'-bipyridine] de ruthénium, désigné par la suite Ru(DMA-bpy)₂(TEAM-bpy) et ses sels.
- bis[4,4'-bis(triéthylammoniumméthyl) 2,2'bipyridine]mono(4,4'-bisamino)2,2'-bipyridine d'Osmium, désigné par la suite Os(TEAM-bpy)₂(DA,-bpy) et ses sels.
- tris[4,4'-bis(triéthylammoniumméthyl)-2,2'-bipyridine] d'Osmium, désigné par la suite Os(TEAM-bpy)_{3,} et ses sels.
- tris[4,4'-bis(triéthylammoniumméthyl)-2,2'-bipyridine] de fer, désigné par la suite Fe(TEAM-bpy)₃, et ses sels.

6. Complexe selon les revendications 1 à 3, caractérisé en ce que -N(R₁R₂R₃) représente le group N-pyridino, et correspond à la formule générale IV 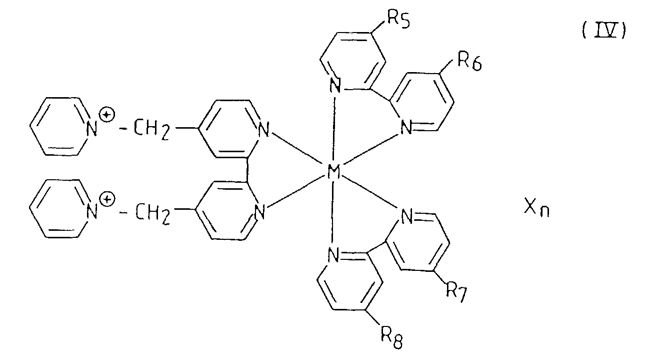 dans laquelle R₅, R₆, R₇, R₈, X et n ont les mêmes significations que celles indiquées pour la formule I.

7. Complexe selon la revendication 6, caractérisé en ce qu'il correspond à l'un des composés de formule
- bis [4,4'-bisamino-2,2'-bipyridine]-mono[4,4'-bis(N-pyridiniumméthyl)-2,2' bipyridine] d'Osmium, désigné par la suite Os(DA-bpy)₂(NPM-bpy) et ses sels.
- bis [4,4'-bisamino-2,2'-bipyridine]- mono[4,4'-bis(N-pyridiniumméthyl)2,2'-bipyridine] de ruthénium, désigné par la suite Ru(DA-bpy)₂(NPM-bpy) et ses sels.
- bis[4,4'-bis (N-pyridiniumméthyl)-2,2'bipyridine mono(4,4'-bisamino-2,2'-bipyridine) d'Osmium, désigné par la suite Os(NPM-bpy)₂(DA-bpy) et ses sels.
- tris[4,4'-bis(N-pyridiniumméthyl)-2,2'-bipyridine] d'Osmium, désigné par la suite Os(NPM-bpy)₃ et ses sels.
- tris[4,4'-bis(N-pyridiniumméthyl)-2,2'-bipyridine] de fer, désigné par la suite Fe(TEAM-bpy)₃ et ses sels.

8. Complexe selon les revendications 1 à 3, caractérisé en ce que le métal de transition est l'Osmium.

9. Complexe selon les revendications 1 à 3, caractérisé en ce que le métal de transition est le ruthénium.

10. Complexe selon les revendications 1 à 3, caractérisé en ce que le métal de transition est le fer.

11. Complexe selon les revendications 1 à 10, caractérisé en ce que le sel est un chlorure ou un héxyfluorophosphate.

12. Composé bipyridine substitué par au moins un groupe ammonium quaternaire pour la synthèse des complexes selon les revendications 1 à 3, caractérisé en ce qu'il correspond à la formule générale II 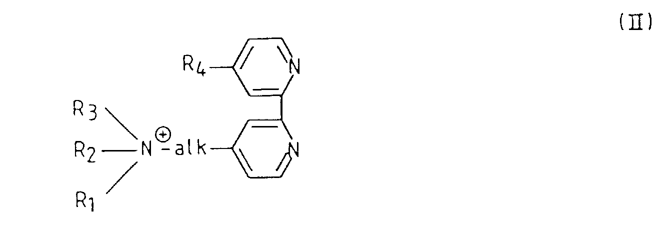 dans laquelle R₁, R₂, R₃, R₄ et "alk" ont les mêmes significations que celles revendiquées par la formule générale I.

13. Procédé de préparation des composés selon les revendications 1 à 12, caractérisé en ce que
- dans une première étape on fait réagir une 4,4'-bis bromoalkyl-2,2' bipyridine sur une amine en excès de formule N(R₁R₂R₃) pour obtenir le composé de formule II et,
- dans une deuxième étape on fait réagir le composé de formule II avec une quantité sensiblement stoechiométrique d'un sel soluble du complexe formé par le métal M et deux ligands bipyridine respectivement substitués par R₅, R₆, R₇ et R₈ les substituants R₁ à R₈, ayant les significations données pour les composés de formule I ainsi obtenus.

14. Procédé selon la revendication 13 pour la préparation des complexes dans lesquels R₄, R₅ et R₆ représentent chacun le groupe -alk-N⁺(R₁R₂R₃), R₁, R₂, R₃, R₆, R₇ et "alk" ayant les significations données pour les complexes de formule (I), caractérisé en ce que la deuxième étape est une première fois appliquée pour la préparation d'un complexe intermédiaire Bis d'un composé de formule (II) avec un métal approprié M.

15. Procédé pour la préparation des complexes selon les revendications 1 à 11 dans lesquels R₄, R₅, R₆, R₇ et R₈ représentent chacun le groupe -alk-N⁺(R₁R₂R₃), R₁, R₂, R₃ et "alk" ayant les significations données pour les composés de formule I, caractérisé en ce qu'on chauffe à reflux de solvant une solution contenant dans des proportions sensiblement stoechiométriques le composé de formule II et un sel soluble du métal M.

16. Application des complexes selon les revendications 1 à 11, caractérisée en ce qu'ils interviennent comme médiateurs dans une réaction redox pour le dosage d'un composant en solution en présence d'une enzyme spécifique dudit composant.

17. Application selon la revendication 16, caractérisée en ce que l'enzyme spécifique est le glucose oxydase GOD et que le composant en solution est le glucose.

18. Application selon la revendication 17, caractérisée en ce que le complexe médiateur est le bis[4,4'-bis(aminométhyl)-2,2'-bipyridine]mono[4,4'-bis(triéthylammoniumméthyl)-2,2'-bipyridine] d'osmium ou l'un de ses sels.

## Patentansprüche

1. Übergangsmetallkomplex mit drei zweifach gezahnten Liganden, von denen wenigstens einer in Position 4,4' durch wenigstens eine quaternäre Ammoniumalkylgruppe substituiert ist, dadurch gekennzeichnet, daß er der allgemeinen Formel I 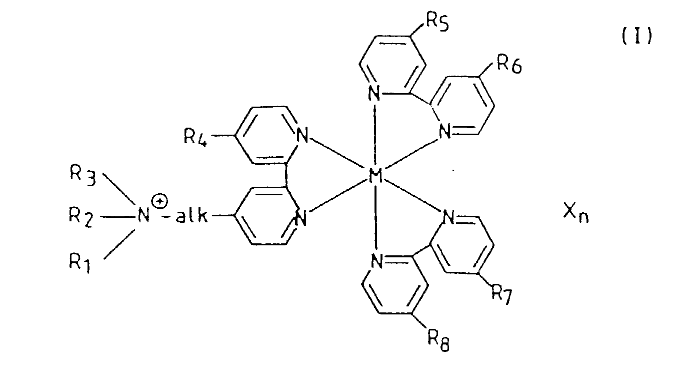 entspricht, in der R₁, R₂ und R₃, die identisch oder unterschiedlich sein können, getrennt ein lineares oder verzweigtes Alkylradikal mit 1 bis 5 Kohlenstoffatomen darstellen oder insgesamt mit dem Stickstoffatom, an das sie gebunden sind, ein heterozyklisches Radikal mit 5 bis 7 Kohlenstoffatomen darstellen; "alk" ein lineares oder verzweigtes Alkylenradikal mit 1 bis 5 Kohlenstoffatomen; R₄ bis R₈, die identisch oder unterschiedlich sein können, ein Wasserstoffatom, eine Hydroxy-, Alkoxy-, Aryloxy-, primäre, sekundäre und tertiäre Aminogruppe oder die Gruppe -alk-N±(R₁R₂R₃) darstellen, in denen "alk", R₁, R₂, R₃ die vorstehenden Bedeutungen haben; M ein Übergangsmetall, wie Eisen, Ruthenium oder Osmium, darstellt, X ein Anion, wie Cl⁻, PF₆⁻, Br⁻, BF₄⁻, und n eine ganze Zahl entsprechend der Anzahl der positiven Ladungen von Liganden und des Übergangsmetalls darstellen.

2. Komplex nach Anspruch 1, dadurch gekennzeichnet, daß das Alkylenradikal "alk" das Radikal Methylen - CH₂ - darstellt.

3. Komplex nach Anspruch 1 und 2, dadurch gekennzeichnet, daß R4 die Gruppe -N⁺ (R₁R₂R₃) darstellt.

4. Komplex nach den Ansprüchen 1 bis 3, dadurch gekennzeichnet, daß -N⁺ (R₁R₂R₃) die Gruppe Triethylammonium darstellt und der allgemeinen Formel III 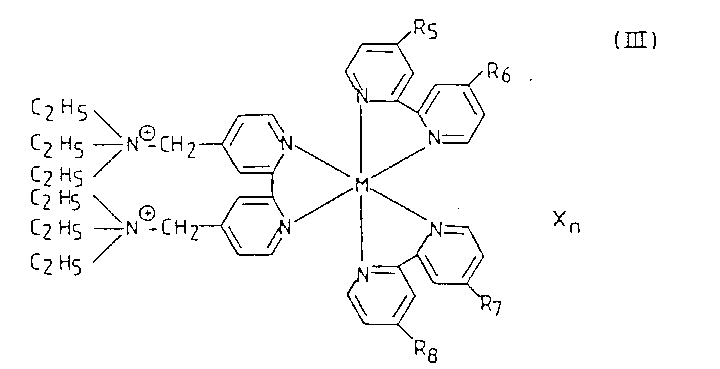 entspricht, in der R₅, R₆, R₇, R₈, X und n die gleichen Bedeutungen wie zur allgemeinen Formel I angegeben besitzen.

5. Komplex nach Anspruch 4, dadurch gekennzeichnet, daß er einer von Verbindungen der Formel
- Bis[4,4'-bisamino-2,2'bipyridin]-mono-[4,4'-bis(triethylammoniumethyl) -2,2'bipyridin] von Osmium, bezeichnet als die Folge Os(DA-bpy)₂(TEAM-bpy), und dessen Salzen,
- Bis[4,4'-bisamino-2,2'-bipyridin]-mono-[4,4'-bis(triethylammoniumethyl) -2,2'bipyridin] von Ruthenium, bezeichnet als die Folge Ru(DA-bpy)₂(TEAM-bpy), und seinen Salzen,
- Bis[4,4'-bis(dimethylamino)-2,2'bipyridin]-mono-[4,4'-bis(triethylaminomethyl)-2,2'-bipyridin] von Ruthenium, bezeichnet als die Folge Ru(DMA-bpy)₂ (TEAM-bpy), und dessen Salzen,
- Bis[4,4'-bis(triethylammoniumethyl)2,2'bipyridin]-mono-(4,4'-bisamino) 2,2'-bipyridin von Osmium, bezeichnet als die Folge Os(TEAM-bpy)₂(DA-bpy), und seinen Salzen,
- Tris[4,4'-bis(triethylammoniumethyl)-2,2'-bipyridin] von Osmium, bezeichnet durch die Folge Os(TEAM-bpy)₃, und seinen Salzen,
- Tris[4,4'-bis(triethylammoniumethyl)-2,2'-bipyridin] von Eisen, bezeichnet durch die Folge Fe(TEAM-bpy)₃, und seinen Salzen, entspricht.

6. Komplex nach den Ansprüchen 1 bis 3, dadurch gekennzeichnet, daß -N (R₁R₂R₃) die N-Pyridin-Gruppe repräsentiert und der allgemeinen Formel IV 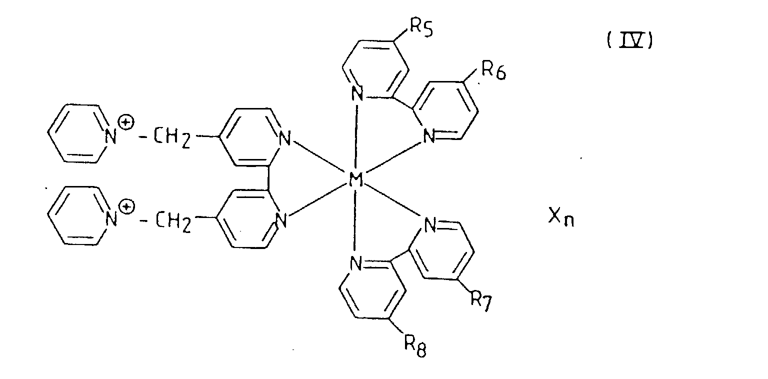 entspricht, in der R₅, R₆, R₇, R₈, X und n die gleichen Bedeutungen wie in bezug auf die Formel I angegeben besitzen.

7. Komplex nach Anspruch 6, dadurch gekennzeichnet, daß er einer der formelmäßigen Zusammensetzungen
- Bis[4,4'-bisamino-2,2'-bipyridin]-mono-[4,4'-bis(N-pyridiniummethyl)-2,2'bipyridin] von Osmium, bezeichnet durch die Folge Os(DA-bpy)₂(NPM-bpy), und seinen Salzen,
- Bis[4,4'-bisamino-2,2'-bipyridin]-mono-[4,4'-bis(N-pyridiniummethyl) 2,2'-bipyridin] von Ruthenium, bezeichnet durch die Folge Ru(DA-bpy)₂(NPM-bpy), und seinen Salzen,
- Bis[4,4'-bis(N-pyridiniummethyl)-2,2'bipyridin-mono-(4,4'-bisamino-2,2'-bipyridin) von Osmium, bezeichnet durch die Folge Os(NPM-bpy)₂(DA-bpy), und seinen Salzen,
- Tris[4,4'-bis(N-pyridiniummethyl)-2,2'-bipyridin] von Osmium, bezeichnet durch die Folge Os(NPM-bpy)₃, und seinen Salzen,
- Tris [4,4'-bis(N-pyridiniummethyl)-2,2'-bipyridin] von Eisen, bezeichnet durch die Folge Fe(TEAM-bpy)₃, und seinen Salzen, entspricht.

8. Komplex nach den Ansprüchen 1 bis 3, dadurch gekennzeichnet, daß das Übergangsmetall Osmium ist.

9. Komplex nach den Ansprüchen 1 bis 3, dadurch gekennzeichnet, daß das Übergangsmetall Ruthenium ist.

10. Komplex nach den Ansprüchen 1 bis 3, dadurch gekennzeichnet, daß das Übergangsmetall Eisen ist.

11. Komplex nach den Ansprüchen 1 bis 10, dadurch gekennzeichnet, daß das Salz ein Chlorid oder ein Hexafluorphosphat ist.

12. Bipyridinverbindung, substituiert durch wenigstens eine quaternäre Ammoniumgruppe zur Synthese von Komplexen gemäß den Ansprüchen 1 bis 3, dadurch gekennzeichnet, daß sie der allgemeinen Formel II 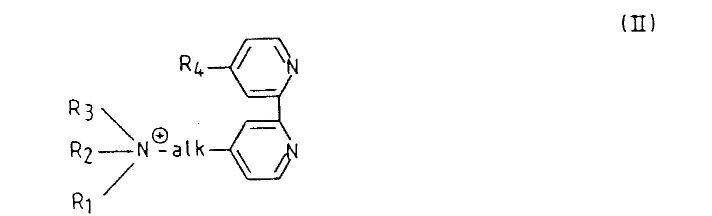 entspricht, in der R₁, R₂, R₃, R₄ und "alk" die gleichen Bedeutungen wie für die allgemeine Formel I beansprucht besitzen.

13. Verfahren zur Herstellung von Verbindungen nach den Ansprüchen 1 bis 12, dadurch gekennzeichnet, daß
- man in einem ersten Schritt ein 4,4'-Bis-bromalkyl-2,2'-bipyridin auf ein Amin im Überschuß von der Formel N (R₁R₂R₃) zum Erhalten der Verbindung der Formel II reagieren läßt und
- man in einem zweiten Schritt die Verbindung der Formel II mit einer im wesentlichen stöchiometrischen Menge eines lösbaren Salzes des durch das Metall M und zwei Bipyridin-Liganden gebildeten Komplexes reagieren läßt, die entsprechend durch R₅, R₆, R₇ und R₈ substituiert sind, wobei die Substituenten R₁ bis R₈ die für die auf diese Weise erhaltenen Verbindungen der Formel I angegebenen Bedeutungen besitzen.

14. Verfahren nach Anspruch 13 zur Herstellung von Komplexen, in denen R₄, R₅ und R₆ jeweils die Gruppe -alk-N⁺(R₁R₂R₃) darstellen, R₁, R₂, R₃, R₆, R₇ und "alk" die für die Komplexe der Formel (I) angegebenen Bedeutungen besitzen, dadurch gekennzeichnet, daß der zweite Schritt ein erstes Mal zur Herstellung eines intermediären Bis-Komplexes einer Verbindung der Formel (II) mit einem geeigneten Metall M angewendet wird.

15. Verfahren zur Herstellung von Komplexen gemäß den Ansprüchen 1 bis 11, in denen R₄, R₅, R₆, R₇ und R₈ jeweils die Gruppe -alk-N⁺(R₁R₂R₃) repräsentieren, wobei R₁, R₂, R₃ und "alk" die für die Verbindungen der Formel I angegebenen Bedeutungen besitzen, dadurch gekennzeichnet, daß man im Lösungsmittelrückfluß eine Lösung erwärmt, die in im wesentlichen stöchiometrischen Anteilen die Verbindung der Formel II und ein lösbares Salz des Metalls M enthält.

16. Verwendung von Komplexen gemäß den Ansprüchen 1 bis 11, dadurch gekennzeichnet, daß sie als Beschleuniger in einer Redox-Reaktion für das Dosieren eines in Lösung befindlichen Bestandteils in Anwesenheit eines spezifischen Enzyms dieses Bestandteils auftreten.

17. Verwendung nach Anspruch 16, dadurch gekennzeichnet, daß das spezifische Enzym die Glucose-Oxydase-GOD und daß der Bestandteil in Lösung die Glucose ist.

18. Verwendung nach Anspruch 17, dadurch gekennzeichnet, daß der Beschleunigerkomplex das Bis[4,4'-bis(aminomethyl)-2,2'-bipyridin]-mono-[4,4'-bis(triethylammoniummethyl)-2,2'-bipyridin] von Osmium oder eines seiner Salze ist.

## Claims

1. A transition metal complex having three bidentate ligands at least one of which is substituted in the 4,4' position by at least one quaternary ammonium alkyl group, characterised in that is has the general formula 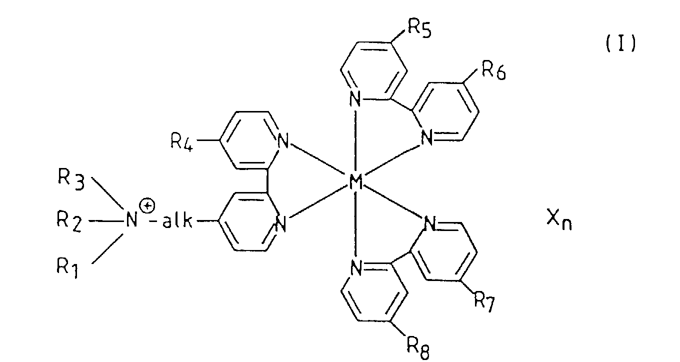 wherein R₁, R₂ and R₃ may be the same or different and each represents a straight chain or branched chain alkyl group having 1 to 5 carbon atoms or taken together with the adjacent nitrogen atom represent a heterocyclic radical having 5 to 7 carbon atoms; "alk" represents a straight chain or branched alkylene radical having 1 to 5 carbon atoms; R₄ to R₈, which may be the same or different, represent hydrogen, or a hydroxy, alkoxy, aryloxy or primary, secondary or tertiary amino group or the group -alk-N^{±}(R₁R₂R₃) wherein "alk", R₁, R₂ and R₃ have the meanings given above; M represents a transition metal such as iron, ruthenium or osmium; X represents an anion such as Cl⁻ , PF₆⁻, Br⁻, BF₄⁻ and n is an integer corresponding to the number of positive charges of the ligands and of the transition metal.

2. A complex according to claim 1, characterised in that the alkylene radical "alk" represents a methylene radical -CH₂-.

3. A complex according to claim 1 or 2, characterised in that R₄ represents the group -N(R₁R₂R₃).

4. A complex according to claims 1 to 3, characterised in that -N(R₁R₂R₃) represents the triethylammonium group and corresponds to the general formula III 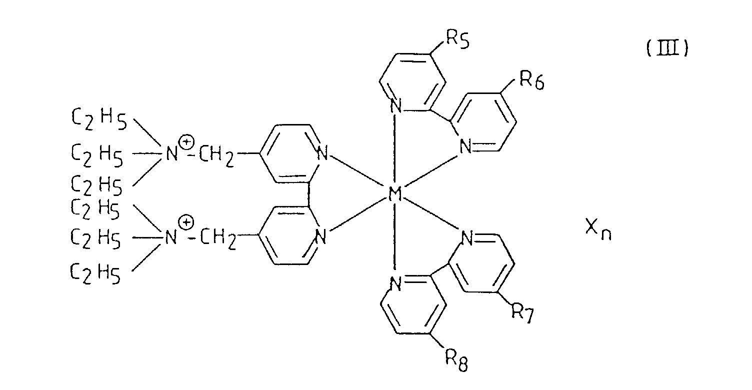 wherein R₅, R₆, R₇, R₈, X and n have the same meanings as those given for the general formula I.

5. A complex according to claim 4, characterised in that it corresponds to one of the compounds of formula
- osmium bis[4,4'-bisamino-2,2'-bipyridine]-mono[4,4'-bis(triethylammoniummethyl)-2,2'-bipyridine, hereinafter referred to as Os(DA-bpy)₂(TEAM-bpy) and salts thereof,
- ruthenium bis[4,4'-bisamino-2,2'-bipyridine]-mono[4,4'-bis(triethylammoniummethyl)-2,2'-bipyridine, hereinafter referred to as Ru(DA-bpy)₂(TEAM-bpy) and salts thereof,
- ruthenium bis[4,4'-bis(dimethylamino-2,2'-bipyridine]- mono[4,4'-bis(triethylaminomethyl)-2,2'-bipyridine], hereinafter referred to as Ru(DMA-bpy)₂(TEAM-bpy) and salts thereof,
- osmium bis[4,4'-bis(triethylammoniummethyl) 2,2'-bipyridine]mono[4,4'-bisamino)2,2'-bipyridine, hereinafter referred to as Os(TEAM-bpy)₂(DA-bpy) and salts thereof,
- osmium tris[4,4'-bis(triethylammoniummethyl)-2,2'-bipyridine], hereinafter referred to as Os(TEAM-bpy)₃, and salts thereof,
- iron tris[4,4'-bis(triethylammoniummethyl)-2,2'-bipyridine], hereinafter referred to as Fe(TEAM-bpy)₃, and salts thereof.

6. A complex according to claims 1 to 3 characterised in that -N(R₁R₂R₃) represents the N-pyridino group and has the general formula IV 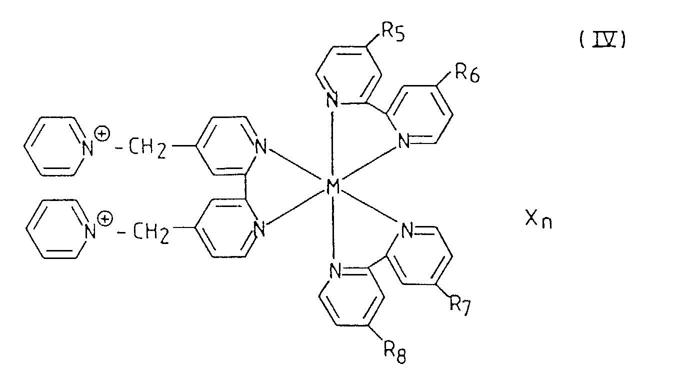 wherein R₅, R₆, R₇, R₈, X and n have the same meanings as those given for formula I.

7. A complex according to claim 6, characterised in that it corresponds to one of the compounds of formula
- osmium bis[4,4'-bisamino-2,2'-bipyridine]-mono[4,4'-bis(N-pyridiniummethyl)-2,2'-bipyridine], hereinafter referred to as Os(DA-bpy)₂(NPM-bpy) and salts thereof,
- ruthenium bis[4,4'-bisamino-2,2'-bipyridine]-mono[4,4'-bis(N-pyridiniummethyl)-2,2'-bipyridine], hereinafter referred to as Ru(DA-bpy)₂(NPM-bpy) and salts thereof,
- osmium bis[4,4'-bis(N-pyridiniummethyl)-2,2'-bipyridine]mono[4,4'-bisamino-2,2'-bipyridine), hereinafter referred to as Os(MPM-bpy)₂(DA-bpy) and salts thereof,
- osmium tris [4,4'-bis(N-pyridiniummethyl) 2,2'-bipyridine] hereinafter referred to as Os(NPM-bpy)₃ and salts thereof,
- iron tris[4,4'-bis(N-pyridiniummethyl)-2,2'-bipyridine], hereinafter referred to as Fe(TEAM-bpy)₃, and salts thereof.

8. A complex according to claims 1 to 3, characterised in that the transition metal is osmium.

9. A complex according to claims 1 to 3, characterised in that the transition metal is ruthenium.

10. A complex according to claims 1 to 3, characterised in that the transition metal is iron.

11. A complex according to claims 1 to 10, characterised in that the salt is a chloride or a hexafluorophosphate.

12. A bipyridine compound substituted by at least one quaternary ammonium group for the synthesis of complexes according to claims 1 to 3, characterised in that it corresponds to the general formula II 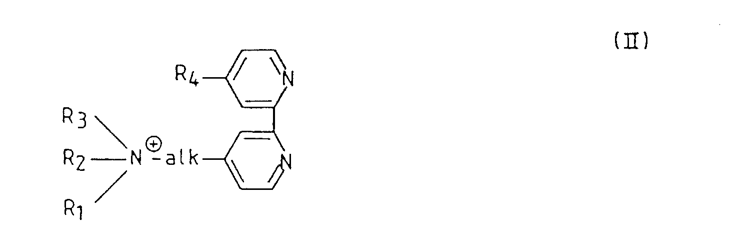 wherein R₁, R₂, R₃, R₄ and "alk" have the same meanings as those given for the general formula I.

13. A process for the preparation of compounds according to claims 1 to 12, characterised in that
- in a first step a 4,4'-bis bromoalkyl-2,2'-bipyridine is reacted with an amine in excess of a compound of formula N(R₁R₂R₃) to yield the compound of formula II, and,
- in a second step the compound of formula II is reacted with a substantially stoichiometric amount of a soluble salt of a complex formed by the metal M and two bipyridine ligands substituted respectively by R₅, R₆, R₇ and R₈, the substituents R₁ to R₈ having the meanings given for the compounds of formula I so obtained.

14. A process according to claim 13 for the preparation of complexes wherein R₄, R₅ and R₆ each represent the group -alk-N⁺(R₁R₂R₃), R₁, R₂, R₃, R₆, R₇ and "alk" having the meanings given for the complexes of formula (I), characterised in that the second step is applied a first time for the preparation of an intermediate bis complex of a compound of formula (II) with an appropriate metal M.

15. A process for the preparation of complexes according to claims 1 to 11 wherein R₄, R₅, R₆, R₇ and R₈ each represent the group -alk-N⁺(R₁R₂R₃), R₁, R₂, R₃ and "alk" having the meanings given for the compounds of formula (I), characterised in that a solution containing substantially stoichiometric proportions of the compound of formula II and a soluble salt of the metal M is refluxed.

16. The use of complexes according to claims 1 to 11, characterised in that they intervene as mediators in a redox reaction for analysing a compound in solution in the presence of an enzyme specific for the said compound.

17. The use according to claim 16, characterised in that the specific enzyme is glucose oxidase GOD and the compound in solution is glucose.

18. The use according to claim 17, characterised in that the mediator complex is osmium bis[4,4'-bis(aminomethyl)-2,2'-bipyridine]mono[4,4'-bis(triethylammoniummethyl)-2,2'-bipyridine]or a salt thereof.
